# EUROPÄISCHE PATENTSCHRIFT

(11) **EP 3 634 326 B1**
(45) Veröffentlichungstag und Bekanntmachung des Hinweises auf die Patenterteilung: **06.11.2024**
(21) Anmeldenummer: 18746084.5
(22) Anmeldetag: 05.06.2018
(51) Int. Cl.: A61F 9/007

(54) **HOHLNADEL FÜR EIN AUGENCHIRURGISCHES INSTRUMENT**
HOLLOW NEEDLE FOR AN OPHTHALMIC SURGICAL INSTRUMENT
AIGUILLE CREUSE POUR UN INSTRUMENT DE CHIRURGIE OCULAIRE

(30) Priorität: 07.06.2017 DE 102017209595
(43) Veröffentlichungstag der Anmeldung: 15.04.2020
(73) Patentinhaber: Geuder AG, 69126 Heidelberg (DE)
(72) Erfinder: ENGEL, Stefan, 69126 Heidelberg (DE)
(74) Vertreter: Ullrich & Naumann PartG mbB
(86) Internationale Anmeldenummer: PCT/DE2018/200056
(87) Internationale Veröffentlichungsnummer: WO 2018/224100

(56) Entgegenhaltungen:
- EP-A1- 2 582 336
- EP-B1- 2 582 336
- DE-A1- 102015 207 150
- US-A- 5 741 226
- US-A1- 2009 099 536
- US-A1- 2017 027 753

## Beschreibung

Die vorliegende Erfindung betrifft eine Hohlnadel für ein augenchirurgisches Instrument zur in-vivo-Zertrümmerung und Entfernung organischer Linsen mittels Ultraschall, mit einem am proximalen Ende ausgebildeten Anschlussbereich zum Ankoppeln an das Instrument und einer am distalen Ende ausgebildeten Nadelspitze mit mindestens einer Wirkfläche zum Zertrümmern des Linsenmaterials, wobei sich in Axialrichtung ein Absaugkanal zum Absaugen von Linsentrümmern durch die Hohlnadel hindurch erstreckt, dessen mindestens eine Öffnung durch eine Wirkfläche begrenzt ist.

Hohlnadeln der hier in Rede stehenden Art sind aus der Praxis hinlänglich bekannt. Eine entsprechende Hohlnadel für die Ophthalmologie ist in der US 6,074,396 A gezeigt. Dabei weist der Absaugkanal eine Öffnung am distalen Ende der Hohlnadel auf.

Des Weiteren zeigt auch die DE 196 46 881 C1 eine Hohlnadel zur Anwendung in der Ophthalmologie. Im Konkreten handelt es sich dabei um eine Hohlnadel für ein augenchirurgisches Instrument zur in-vivo- Zertrümmerung von Linsen durch Hochfrequenzbetätigung der Hohlnadel, wobei die Hohlnadel gleichzeitig zum Absaugen von Linsentrümmern durch einen inneren Absaugkanal dient. Die Hohlnadel umfasst am distalen Ende ein ringförmig ausgebildetes Stirnende, das die Öffnung des Absaugkanals bildet.

Ultraschallbetätigte Hohlnadeln der gattungsbildenden Art werden bei Kataraktoperationen in der Augenchirurgie verwendet. Das distale, freie Ende der Hohlnadel wird in eine hochfrequente Axialbewegung versetzt und unmittelbar an den Katarakt herangeführt. Vom ringförmigen Stirnende werden Ultraschallwellen zur Emulsifikation des Gewebes abgestrahlt. Abgetrennte Linsenteile bzw. Linsentrümmer werden durch die Hohlnadel hindurch gemeinsam mit einer dem Auge zugeführten Spülflüssigkeit abgeführt.

Die zum Abstrahlen von Ultraschallwellen dienende Wirkfläche kann dadurch vergrößert werden, dass die Stirnseite gezahnt ausgestaltet wird. Dies führt zu einer Verstärkung des emittierten Ultraschallfeldes und verbessert die Effizienz des Instruments bzw. der Hohlnadel.

Aus der US 5,741,226 ist eine Hohlnadel für ein augenchirurgisches Instrument zur in-vivo-Zertrümmerung und Entfernung organischer Linsen mittels Ultraschall mit geschlossener Nadelspitze und seitlichen Wirkflächen bekannt.

Die bekannten Hohlnadeln sind jedoch in der Praxis durch die eine Wirkfläche und die eine Öffnung begrenzt. Des Weiteren sind sie insoweit problematisch, dass ein Teilen bzw. Zerschneiden der Linse ausschließlich durch eine Kreisringfläche an der Nadelspitze aufgrund des ringförmig ausgebildeten Stirnendes realisierbar ist.

Der vorliegenden Erfindung liegt daher die Aufgabe zugrunde, eine Hohlnadel für ein augenchirurgisches Instrument der gattungsbildenden Art derart auszugestalten und weiterzubilden, dass mit konstruktiv einfachen Mitteln in kurzer Zeit eine präzise und effektive Zertrümmerung organischer Linsen ermöglicht ist.

Erfindungsgemäß ist erkannt worden, dass es zur optimalen Bearbeitung des Katarakts nicht erforderlich ist den Wirkbereich sowie den durch den Wirkbereich begrenzte Öffnung des Absaugkanals am distalen Ende einer Hohlnadel vorzusehen und dort das emittiere Ultraschallfeld zu maximieren. Ganz im Gegenteil ist erkannt worden, dass die in-vivo-Zertrümmerung und Entfernung organischer Linsen mittels Ultraschall in verblüffend einfacher Weise dadurch verbessert werden kann, das distale Ende geschlossen auszuführen und mit einer Schneidfläche zum Zerschneiden bzw. Zerteilen des Linsenmaterials zu versehen und mindestens einen Wirkbereich samt Öffnung seitlich nahe der Nadelspitze vorzusehen. In anderen Worten wird die Linse durch eine scharfe Schneidfläche an der Spitze der Hohlnadel geteilt bzw. zerschnitten. Der aufgrund des Ultraschalls entstehende Hub in Axialrichtung und die dadurch entstehende Kavitation an der geschlossenen Nadelspitze verstärken den Effekt des Zerschneidens. Die mindestens eine seitliche Wirkfläche strahlt Ultraschallwellen in Richtung der zerschnittenen bzw. zerteilten Linse und zertrümmert diese. Entstehende Kavitationen verstärken den Effekt. Durch die seitliche Anordnung der mindestens einen Wirkfläche kann diese in Axial- und/oder Umfangrichtung der Hohlnadel vergrößert und damit das Ultraschallfeld und die Kavitation maximiert werden. Die zertrümmerten Linsenfragmente werden über die mindestens eine, durch die seitliche Wirkfläche begrenzte Öffnung abgesaugt.

Folglich ist mit der beanspruchten Erfindung eine Hohlnadel gemäß Anspruch 1 realisiert, die mit konstruktiv einfachen Mitteln in kurzer Zeit eine präzise und effektive Zertrümmerung organischer Linsen ermöglicht.

Von ganz besonderem Vorteil ist es, wenn die Schneidfläche als Schneidkante im Sinne einer scharfen Messerschneide ausgeführt ist, um den Linsenkern leichter zu zerteilen bzw. zu zerschneiden.

Zur weiteren Verbesserung der Schneideigenschaft ist es von besonderem Vorteil, wenn die Schneidkante schräg zu der Axialrichtung der Hohlnadel verläuft.

In besonders vorteilhafter Weise weist die Schneidkante einen Wellenschliff auf. Dies verbessert den Effekt des Schneidens des Linsenkerns abermals, wobei die Schneidkante im Sinne eines Sägemessers oder einer Hubsäge fungiert. Auch andere Klingenschliffe sind denkbar.

Zur Verringerung der Bearbeitungszeit, d.h. der Zeit für das Zertrümmern des Linsenmaterials und das Absaugen von Linsentrümmern, ist es von ganz besonderem Vorteil, wenn die Hohlnadel zwei seitliche, jeweils eine Öffnung begrenzende Wirkflächen, aufweist. Durch die Nutzung zweier Wirkflächen kann das emittierte Ultraschallfeld vergrößert und die Effizienz des Instruments bzw. der Hohlnadel verbessert, nämlich. die Wirkzeit des Ultraschallfeldes verringert werden. Auch können die zertrümmerten Linsenfragmente durch zwei Öffnungen schneller abgetragen und abgesaugt werden. Bei zwei Wirkflächen ist es von Vorteil, wenn diese sich im Wesentlichen gegenüberliegend angeordnet sind.

Des Weiteren ist es von besonderem Vorteil, wenn die Wirkflächen schräg zu der Axialrichtung verlaufen, so dass sich die Nadelspitze verjüngt. Dies ermöglicht einen optimalen Übergang zwischen dem Bereich der Wirkfläche und der Schneidkante. Zudem lassen sich Kavitationen optimal nach schräg vorne, d.h. in Richtung des zu zertrümmernden Linsenmaterials richten. Ein weiterer Vorteil einer schrägen Anordnung der Wirkflächen besteht darin, dass die Wirkflächen vergrößert und dadurch die Effektivität der Hohlnadel abermals erhöht werden kann.

Bei mehreren Wirkflächen können diese in gleichen oder unterschiedlichen Winkeln gegenüber der Axialrichtung ausgebildet sein, abhängig von der gewünschten Geometrie der Hohlnadel bzw. der Nadelspitze.

Des Weiteren ist es denkbar, dass die Wirkflächen mindestens zwei in unterschiedlichen Winkeln gegenüber der Axialrichtung verlaufende Wirkflächenbereiche aufweisen. Hierdurch lassen sich Kavitationsrichtung und -Stärke sowie die Aspirations- und Abtragungsrichtung beeinflussen und optimieren.

In weiter vorteilhafter Weise ist die Wirkfläche durch eine äußere Kante abgegrenzt, wobei die äußere Kante angefast, stumpf oder scharf ausgebildet sein kann. Eine angefaste bzw. stumpfe Ausbildung der Kante dient zur Vermeidung von Verletzungen des umliegenden Gewebes. Durch eine scharfe Ausgestaltung der äußeren Kante ist eine besonders effektive Zertrümmerung der Linse möglich.

Des Weiteren sei an dieser Stelle angemerkt, dass zur weiteren Optimierung der Effektivität der Hohlnadel sowohl die Form als auch die Dimensionierung der Öffnungen zum Absaugkanal und der Wirkflächen variierbar sind. Denkbar ist eine im Westlichen ovale und/oder mehreckige Ausgestaltung und/oder Einschnitte, bzw. zahnartige Strukturen.

Es gibt nun verschiedene Möglichkeiten die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein erstes Ausführungsbeispiel einer erfindungsgemäßen Hohlnadelmit, wobei die Schneidkante einen Wellenschnitt aufweist,
- Fig. 2: in einer schematischen Ansicht, vergrößert, die Nadelspitze der Hohlnadel aus Fig. 1,
- Fig. 3: in einer schematischen Ansicht die Hohlnadel aus Fig. 1 um 90° rotiert,
- Fig. 4: in einer schematischen Ansicht ein zweites Ausführungsbeispiel einer erfindungsgemäßen Hohlnadel mit gerader Schneidkante,
- Fig. 5: in einer schematischen Ansicht, vergrößert, die Nadelspitze der Hohlnadel gemäß Fig. 4 und
- Fig. 6: in einer schematischen Ansicht die Hohlnadel gemäß Fig. 4 um 90° rotiert.

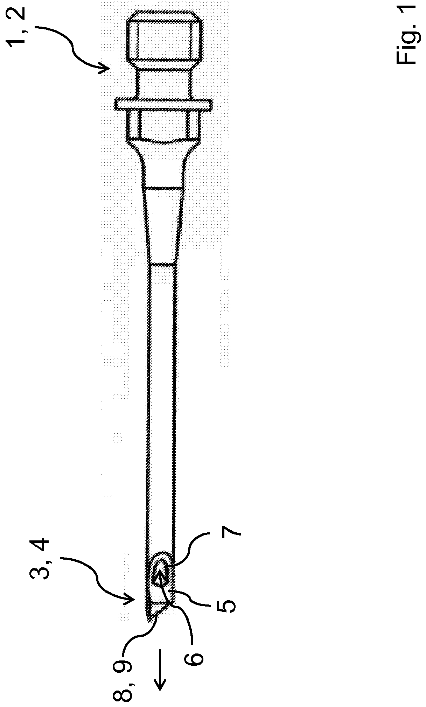

Fig. 1 zeigt in einer schematischen Ansicht ein Ausführungsbeispiel einer erfindungsgemäßen Hohlnadel für ein augenchirurgisches Instrument zur In-vivo-Zertrümmerung organischer Linsen mittels Ultraschall. Die Hohlnadel umfasst einen am proximalen Ende 1 ausgebildeten Anschlussbereich 2 zum Ankoppeln an das Instrument und eine am distalen Ende 3 ausgebildete Nadelspitze 4.

Das distale Ende 3 der Nadelspitze 4 ist geschlossen ausgeführt und weist eine zu der Axialrichtung schräge Schneidkante 8 zum Zerschneiden der Linse auf. Die in Axialrichtung entstehende Kavitation ist durch den geraden durchgezogenen Pfeil symbolisiert. Des Weiteren weist die Schneidkante einen Wellenschliff 9 auf, der den Effekt des Schneidens des Linsenkerns abermals verstärkt.

Unterhalb der Schneidkante bzw. nahe der Nadelspitze 4 sind seitlich, d.h. an der Umfangfläche der Hohlnadel zwei sich gegenüberliegende Wirkflächen 5 zum Zertrümmern des Linsenmaterials vorgesehen, wobei in Fig. 1 nur eine der Wirkflächen 5 zu sehen ist. Durch die Realisierung zweier Wirkflächen 5 kann die Bearbeitungszeit erheblich reduziert, im Vergleich zu Hohlnadeln mit nur einer Wirkfläche und einer Öffnung idealerweise verdoppelt werden.

Durch die Hohlnadel hindurch erstreckt sich in Axialrichtung ein Absaugkanal 6 zum Absaugen von Linsentrümmern, dessen Öffnungen 7 jeweils durch eine Wirkfläche 5 begrenzt sind. Aufgrund zweier Öffnungen 7 zum Absaugen der zertrümmerten Linsenfragmente ist ein schnelleres Abtragen realisierbar als bei aus der Praxis bekannten Hohlnadeln mit nur einer Öffnung am distalen Ende der Hohlnadel.

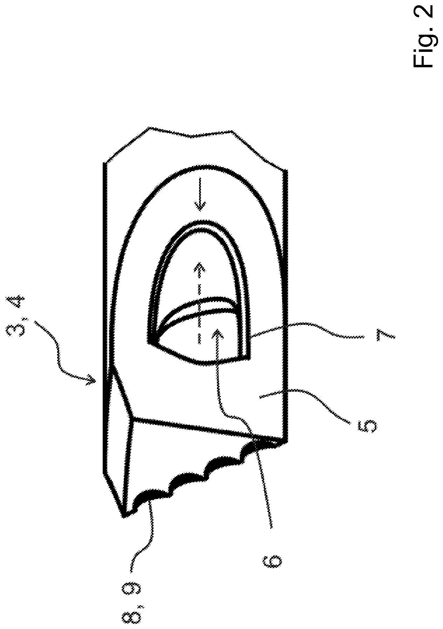

Fig. 2 zeigt in einer schematischen Ansicht, vergrößert, die Nadelspitze 4 der Hohlnadel gemäß Fig. 1. Im Konkreten ist am distalen Ende 3 der Nadelspitze 4 die Schneidkante 8 gezeigt, die einen Wellenschliff 9 aufweist. Die Wirkflächen 5 begrenzen die Öffnungen 7 und sind im Wesentlichen oval ausgeführt. Die beiden Wirkflächen 5 liegen sich gegenüber. Der gerade durchgezogene Pfeil symbolisiert die Kavitationsrichtung, der gerade gestrichelte Pfeil die Aspirations- und Abtragungsrichtung.

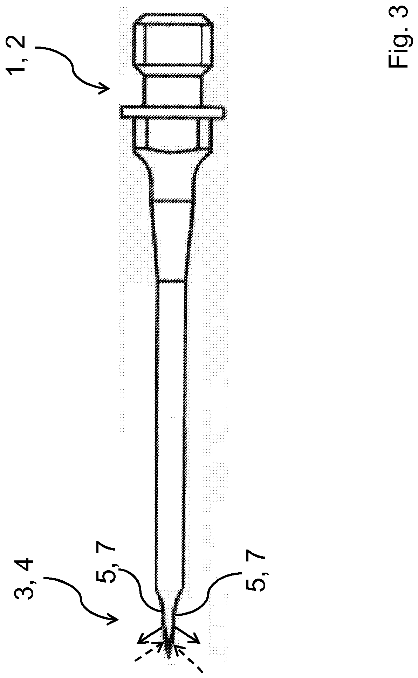

Fig. 3 zeigt in einer schematischen Ansicht die Hohlnadel gemäß Fig. 1 um 90° rotiert. Die Wirkflächen 5 verlaufen schräg zu der Axialrichtung, so dass sich die Nadelspitze 4 verjüngt. Dadurch wird die Kavitation auf den zuvor gespalteten Linsenkern gerichtet. Die Abtragungs- bzw. Aspirationsrichtung ergibt sich genau in umgekehrte Richtung. Auch hier symbolisieren die geraden durchgezogenen Pfeile die Kavitationsrichtung, die geraden gestrichelten Pfeil die Aspirations- und Abtragungsrichtung.

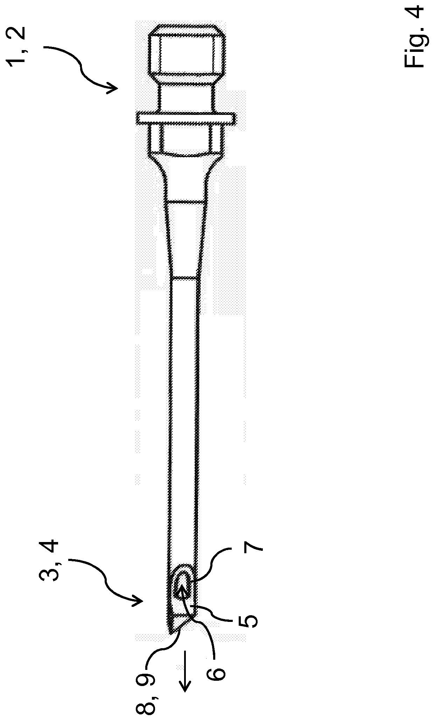

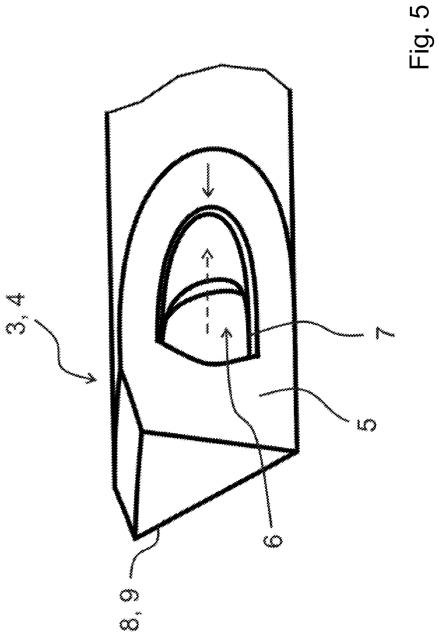

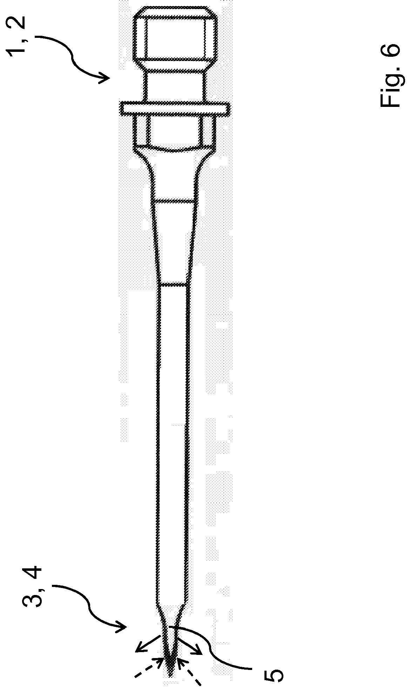

Fig. 4 bis 6 zeigen jeweils in einer schematischen Ansicht ein zweites Ausführungsbeispiel einer erfindungsgemäßen Hohlnadel, wobei sich die Hohlnadel lediglich darin von der Hohlnadel gemäß dem ersten Ausführungsbeispiel unterscheidet, dass die Schneidfläche 8 gerade, d.h. ohne Wellenschliff ausgeführt ist.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Proximales Ende
- 2: Anschlussbereich
- 3: Distales Ende
- 4: Nadelspitze
- 5: Wirkfläche
- 6: Absaugkanal
- 7: Öffnung
- 8: Schneidkante
- 9: Wellenschliff

## Patentansprüche

1. Hohlnadel für ein augenchirurgisches Instrument zur in-vivo-Zertrümmerung und Entfernung organischer Linsen mittels Ultraschall, mit einem am proximalen Ende (1) ausgebildeten Anschlussbereich (2) zum Ankoppeln an das Instrument und einer am distalen Ende (3) ausgebildeten Nadelspitze (4) mit mindestens einer Wirkfläche (5) zum Zertrümmern des Linsenmaterials, wobei sich in Axialrichtung ein Absaugkanal (6) zum Absaugen von Linsentrümmern durch die Hohlnadel hindurch erstreckt, dessen mindestens eine Öffnung (7) durch eine Wirkfläche (5) begrenzt ist, wobei das distale Ende (3) der Nadelspitze (4) geschlossen ausgeführt ist und die mindestens eine Wirkfläche (5) seitlich nahe der Nadelspitze (4) angeordnet ist,
**dadurch gekennzeichnet, dass** die geschlossene Nadelspitze (4) eine scharfe Schneidfläche zum Zerschneiden der Linse aufweist.

2. Hohlnadel nach Anspruch 1, **dadurch gekennzeichnet, dass** die Schneidfläche als Schneidkante (8) ausgeführt ist.

3. Hohlnadel nach Anspruch 2, **dadurch gekennzeichnet, dass** die Schneidkante (8) senkrecht oder schräg zu der Axialrichtung verläuft.

4. Hohlnadel nach Anspruch 2 oder 3, **dadurch gekennzeichnet, dass** die Schneidkante (8) einen Wellenschliff (9) aufweist.

5. Hohlnadel nach einem der Ansprüche 1 bis 4, **dadurch gekennzeichnet, dass** zwei seitliche, jeweils eine Öffnung (7) begrenzende Wirkflächen (5) vorgesehen sind.

6. Hohlnadel nach einem der Ansprüche 1 bis 5, **dadurch gekennzeichnet, dass** die Wirkflächen (5) im Wesentlichen sich gegenüber liegend angeordnet sind.

7. Hohlnadel nach einem der Ansprüche 1 bis 6, **dadurch gekennzeichnet, dass** die Wirkflächen (5) schräg zu der Axialrichtung verlaufen, so dass sich die Nadelspitze (4) verjüngt.

8. Hohlnadel nach einem der Ansprüche 1 bis 7, **dadurch gekennzeichnet, dass** die Wirkflächen (5) in gleichen oder unterschiedlichen Winkeln gegenüber der Axialrichtung ausgebildet sind.

9. Hohlnadel nach einem der Ansprüche 1 bis 8, **dadurch gekennzeichnet, dass** die Wirkflächen (5) mindestens zwei in unterschiedlichen Winkeln gegenüber der Axialrichtung verlaufende Wirkflächenbereiche aufweisen.

10. Hohlnadel nach einem der Ansprüche 1 bis 9, **dadurch gekennzeichnet, dass** die Wirkflächen (5) durch eine äußere Kante begrenzt sind, wobei die äußere Kante angefast, stumpf oder scharf ausgebildet ist.

## Claims

1. Hollow needle for an ophthalmic surgical instrument for in vivo destruction and removal of organic lenses by means of ultrasound, having a connection region (2) which is formed at the proximal end (1) for coupling to the instrument and a needle tip (4) which is formed at the distal end (3) having at least one active face (5) for destroying the lens material, wherein in an axial direction a suction channel (6) for drawing off lens debris extends through the hollow needle, at least one opening (7) of which is delimited by an active face (5), wherein the distal end (3) of the needle tip (4) is configured to be closed and the at least one active face (5) is arranged laterally close to the needle tip (4), **characterised in that** the closed needle tip (4) has a sharp cutting face for cutting the lens.

2. Hollow needle according to claim 1, **characterised in that** the cutting face is in the form of a cutting edge (8).

3. Hollow needle according to claim 2, **characterised in that** the cutting edge (8) extends perpendicularly or obliquely with respect to the axial direction.

4. Hollow needle according to claim 2 or 3, **characterised in that** the cutting edge (8) has a serrated edge (9).

5. Hollow needle according to any one of claims 1 to 4, **characterised in that** two lateral active faces (5) which each delimit an opening (7) are provided.

6. Hollow needle according to any one of claims 1 to 5, **characterised in that** the active faces (5) are arranged substantially opposite each other.

7. Hollow needle according to any one of claims 1 to 6, **characterised in that** the active faces (5) extend obliquely with respect to the axial direction so that the needle tip (4) tapers.

8. Hollow needle according to any one of claims 1 to 7, **characterised in that** the active faces (5) are formed at identical or different angles with respect to the axial direction.

9. Hollow needle according to any one of claims 1 to 8, **characterised in that** the active faces (5) have at least two active face regions which extend at different angles with respect to the axial direction.

10. Hollow needle according to any one of claims 1 to 9, **characterised in that** the active faces (5) are delimited by an outer edge, wherein the outer edge is constructed to be chamfered, blunt or sharp.

## Revendications

1. Aiguille creuse pour un instrument de chirurgie oculaire pour la fragmentation et l'élimination in vivo de lentilles organiques au moyen d'ultrasons, avec une partie de raccordement (2) disposée au niveau de l'extrémité proximale (1), pour le couplage à l'instrument et une pointe d'aiguille (4) disposé au niveau de l'extrémité distale (3), avec au moins une surface active (5) pour la fragmentation du matériau de la lentille, dans laquelle un canal d'aspiration (6), pour l'aspiration des fragments de lentilles, s'étend dans la direction axiale à travers l'aiguille creuse, dont au moins une ouverture (7) est limitée par une surface active (5), dans laquelle l'extrémité distale (3) de la pointe d'aiguille (4) est fermée et l'au moins une surface active (5) est disposée latéralement à proximité de la pointe d'aiguille (4),
**caractérisée en ce que** la pointe d'aiguille fermée (4) comprend une surface de coupe tranchante pour la découpe de la lentille.

2. Aiguille creuse selon la revendication 1, **caractérisée en ce que** la surface de coupe est conçue comme une arête de coupe (8).

3. Aiguille creuse selon la revendication 2, **caractérisée en ce que** l'arête de coupe (8) s'étend perpendiculairement ou de manière inclinée par rapport à la direction axiale.

4. Aiguille creuse selon la revendication 2 ou 3, **caractérisée en ce que** l'arête de coupe (8) présente un bord dentelé (9).

5. Aiguille creuse selon l'une des revendications 1 à 4, **caractérisée en ce que** deux surfaces active (5) latérales limitant chacune une ouverture (7) sont prévues.

6. Aiguille creuse selon l'une des revendications 1 à 5, **caractérisée en ce que** les surfaces actives (5) sont disposées de façon à être globalement en face l'une de l'autre.

7. Aiguille creuse selon l'une des revendications 1 à 6, **caractérisée en ce que** les surfaces actives (5) s'étendent de manière inclinée par rapport à la direction axiale, de sorte que la pointe d'aiguille (4) se rétrécit.

8. Aiguille creuse selon l'une des revendications 1 à 7, **caractérisée en ce que** les surfaces actives (5) sont disposées afin de former des angles identiques ou différents par rapport à la direction axiale.

9. Aiguille creuse selon l'une des revendications 1 à 8, **caractérisée en ce que** les surfaces actives (5) présentent au moins parties de surfaces actives s'étendant avec des angles différents par rapport à la direction axiale.

10. Aiguille creuse selon l'une des revendications 1 à 9, **caractérisée en ce que** les surfaces actives (5) sont limitées par une arête externe, dans laquelle l'arête externe est chanfreinée, obtuse ou tranchante.
